(19) Europäisches Patentamt
European Patent Office
Office européen des brevets

(11) Veröffentlichungsnummer : **0 423 583 B1**

(12) **EUROPÄISCHE PATENTSCHRIFT**

(45) Veröffentlichungstag der Patentschrift :
**16.06.93 Patentblatt 93/24**

(51) Int. Cl.$^5$ : **C07C 50/34,** B01J 19/20

(21) Anmeldenummer : **90119283.1**

(22) Anmeldetag : **08.10.90**

# (54) Verfahren zur Herstellung von Hydroxyanthrachinonen.

(30) Priorität : **20.10.89 DE 3934934**

(43) Veröffentlichungstag der Anmeldung :
**24.04.91 Patentblatt 91/17**

(45) Bekanntmachung des Hinweises auf die Patenterteilung :
**16.06.93 Patentblatt 93/24**

(84) Benannte Vertragsstaaten :
**BE CH DE ES FR IT LI NL**

(56) Entgegenhaltungen :
**DE-A- 2 432 564**
**DE-A- 2 549 372**
**FR-A- 474 487**
**US-A- 2 695 302**

(56) Entgegenhaltungen :
**BERICHTE DER DEUTSCHEN CHEMISCHEN GESELLSCHAFT, Band 64, Nr. 8, 1931, Seiten 2003-2010, Verlag Chemie GmbH, Weinheim, DE; F. MAYER et al.: "Studien in der Reihe des 1-Methyl-anthrachinons"**

(73) Patentinhaber : **BASF Aktiengesellschaft**
**Carl-Bosch-Strasse 38**
**W-6700 Ludwigshafen (DE)**

(72) Erfinder : **Pfister, Jürgen, Dr.**
**Ziegelofenweg 1**
**W-6720 Speyer (DE)**
Erfinder : **Schiessl, Michael**
**Rupprechtstrasse 13**
**W-6701 Altrip (DE)**
Erfinder : **Nachtrab, Rainer**
**An der Froschlache 7**
**W-6700 Ludwigshafen (DE)**

EP 0 423 583 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein neues Verfahren zur Herstellung von 1-Hydroxyanthrachinonen durch Umsetzung von Phthalsäureanhydrid oder dessen Derivaten mit Phenolderivaten in Gegenwart einer Lewis-Säure als Katalysator und gegebenenfalls eines Verdünnungsmittels, wobei man die Umsetzung in einem selbstreinigenden Apparat mit Mischwirkung durchführt.

Aus Houben-Weyl, "Methoden der Organischen Chemie", Band 7/3c, Seite 97, sowie aus Chem. Ber., Band 64, Seiten 2003 bis 2010, 1931, ist es bekannt, Hydrochinon mit 3-Methylphthalsäureanhydrid oder mit 3,6-Dichlorphthalsäureanhydrid in Gegenwart von Aluminiumchlorid als Katalysator und Natriumchlorid als Verdünnungsmittel zu den entsprechenden Anthrachinonen (1-Methyl-5,8-dihydroxyanthrachinon oder 5,8-Dichlor-1,4-dihydroxyanthrachinon) umzusetzen. Nachteilig bei dieser Art der Umsetzung ist der Sachverhalt, daß das Reaktionsgemisch sich während der Umsetzung verfestigt und somit nicht mehr rührbar ist. Für eine Durchführung der Reaktion in größerem Maßstab ist diese Verfahrensweise daher völlig ungeeignet. Dazu kommt noch, daß die Aufarbeitung eines verfestigten Reaktionsgemisches im technischen Maßstab große Probleme mit sich bringt.

Aufgabe der vorliegenden Erfindung war es nun, ein neues Verfahren zur Herstellung von 1-Hydroxyanthrachinonen bereitzustellen, das ebenfalls von Phthalsäureanhydrid oder dessen Derivaten und von Phenolderivaten ausgeht, bei dem aber die oben erwähnten Schwierigkeiten vermieden werden und das außerdem die Zielprodukte in guter Ausbeute und hoher Reinheit bei kurzer Reaktionszeit liefert. Weiterhin sollten die Reaktionspartner in möglichst äquimolarem Verhältnis zueinander zur Reaktion gebracht werden können.

Es wurde nun gefunden, daß die Herstellung von Hydroxyanthrachinonen der Formel I

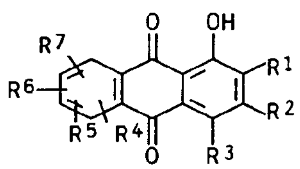

(I),

in der

| | |
|---|---|
| $R^1$ und $R^2$ | gleich oder verschieden sind und unabhängig voneinander jeweils Wasserstoff, Hydroxy, $C_1$-$C_4$-Alkyl oder Halogen oder $R^1$ und $R^2$ zusammen einen anellierten Benzolring, der gegebenenfalls substituiert ist, oder einen anellierten $C_5$-$C_7$-Cycloalkylring, der gegebenenfalls substituiert ist, |
| $R^3$ | Hydroxy, Amino oder Halogen und |
| $R^4$, $R^5$, $R^6$ | und $R^7$ gleich oder verschieden sind und unabhängig voneinander jeweils Wasserstoff, Halogen, Hydroxy, $C_1$-$C_4$-Alkyl oder $C_1$-$C_5$-Alkanoyl bedeuten, |

durch Umsetzung von Phthalsäureanhydriden der Formel II

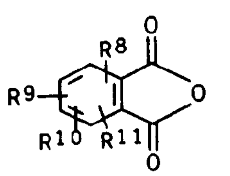

(II),

in der

| | |
|---|---|
| $R^8$, $R^9$, $R^{10}$ | und $R^{11}$ gleich oder verschieden sind und unabhängig voneinander jeweils Wasserstoff, Halogen, $C_1$-$C_4$-Alkyl, $C_1$-$C_5$-Alkanoyl, $C_1$-$C_4$-Alkoxy, $C_1$-$C_5$-Alkanoyloxy oder Sulfato bedeuten, |

mit Phenolderivaten der Formel III

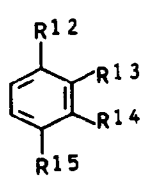

(III),

in der

$R^{12}$ Hydroxy, $C_1$-$C_4$-Alkoxy, $C_1$-$C_5$-Alkanoyloxy oder Sulfato,

$R^{13}$ und $R^{14}$ gleich oder verschieden sind und unabhängig voneinander jeweils Wasserstoff, Hydroxy, $C_1$-$C_4$-Alkoxy, $C_1$-$C_5$-Alkanoyloxy, $C_1$-$C_4$-Alkyl, Halogen oder Sulfato oder $R^{13}$ und $R^{14}$ zusammen einen anellierten Benzolring, der gegebenenfalls substituiert ist, oder einen anellierten $C_5$-$C_7$-Cycloalkylring, der gegebenenfalls substituiert ist, und

$R^{15}$ Hydroxy, $C_1$-$C_4$-Alkoxy, $C_1$-$C_5$-Alkanoyloxy, Sulfato, Halogen oder Mono- oder Di($C_1$-$C_5$-alkanoyl)amino bedeuten,

in Gegenwart einer Lewis-Säure als Katalysator und gegebenenfalls eines Verdünnungsmittels vorteilhaft gelingt, wenn die Umsetzung in einem selbstreinigendem Apparat mit Mischwirkung durchführt.

Alle in den obengenannten Formeln auftretenden Alkylgruppen können sowohl geradkettig als auch verzweigt sein.

Wenn $R^1$ und $R^2$ in Formel I zusammen für einen anellierten Benzolring, der gegebenfalls substituiert ist, oder einen anellierten $C_5$-$C_7$-Cycloalkylring, der gegebenenfalls substituiert ist, stehen, so können als Substituenten z.B. $C_1$-$C_4$-Alkyl, $C_1$-$C_4$-Alkoxy oder Halogen, dabei insbesondere Chlor oder Brom, in Betracht kommen.

Wenn $R^1$ und $R^2$ zusammen für einen anellierten $C_5$-$C_7$-Cycloalkylring stehen, so kommt dafür z.B. der Cyclopentyl-, Cyclohexyl- oder Cycloheptylring in Betracht. Diese Ringe können auch, wie oben ausgeführt, noch weiter substituiert sein.

Reste $R^1$, $R^2$, $R^4$, $R^5$, $R^6$, $R^7$, $R^8$, $R^9$, $R^{10}$, $R^{11}$, $R^{13}$ und $R^{14}$ sind z.B. Methyl, Ethyl, Propyl, Isopropyl, Butyl, Isobutyl oder sec-Butyl.

Reste $R^4$, $R^5$, $R^6$, $R^7$, $R^8$, $R^9$, $R^{10}$, $R^{11}$, $R^{13}$ und $R^{14}$ sind weiterhin, wie auch die Reste $R^3$ und $R^{15}$, z.B. Fluor, Chlor oder Brom.

Reste $R^4$, $R^5$, $R^6$, $R^7$, $R^8$, $R^9$, $R^{10}$ und $R^{11}$ sind weiterhin, z.B. Formyl, Acetyl, Propionyl, Butyryl, Isobutyryl oder Pentanoyl.

Reste $R^8$, $R^9$, $R^{10}$, $R^{11}$, $R^{13}$, $R^{14}$ und $R^{15}$ sind weiterhin, wie auch der Rest $R^{12}$, z.B. Methoxy, Ethoxy, Propoxy, Isopropoxy, Butoxy, Isobutoxy, sec-Butoxy, Formyloxy, Acetyloxy, Propionyloxy, Butyryloxy, Isobutyryloxy oder Pentanoyloxy.

Reste $R^{15}$ sind weiterhin z.B. Mono- oder Diformylamino, Mono- oder Diacetylamino oder Mono- oder Dipropionylamino.

Bevorzugt ist eine Verfahrensweise bei der man Phthalsäureanhydride der Formel II, in der $R^8$, $R^9$, $R^{10}$ und $R^{11}$ gleich oder verschieden sind und unabhängig voneinander jeweils Wasserstoff oder Chlor bedeuten, mit Phenolderivaten der Formel III, in der $R^{12}$ Hydroxy, $C_1$-$C_4$-Alkoxy oder $C_1$-$C_3$-Alkanoyloxy, $R^{13}$ und $R^{14}$ jeweils Wasserstoff und $R^{15}$ Hydroxy, $C_1$-$C_4$-Alkoxy, $C_1$-$C_5$-Alkanoyloxy oder Chlor bedeuten, umsetzt.

Besonders bevorzugt ist eine Verfahrensweise bei der man unsubstituiertes Phthalsäureanhydrid mit Hydrochinon umsetzt. Als Verfahrensprodukt erhält man in diesem Fall Chinizarin (1,4-Dihydroxyanthrachinon).

Das erfindungsgemäße Verfahren wird in Gegenwart einer Lewis-Säure durchgeführt. Für das neue Verfahren geeignete Lewis-Säuren sind z.B. Bortrifluorid, Aluminiumchlorid, Aluminiumbromid, Zinn(IV)chlorid, Antimon(III)chlorid, Antimon(III)bromid, Antimon(V)chlorid, Zinkchlorid, Quecksilber(II)chlorid, Quecksilber(II)bromid, Titan(IV)chlorid, Titan(IV)bromid, Molybdän(IV)bromid, Eisen(III)chlorid oder Eisen(III)bromid.

Bevorzugt sind dabei Aluminiumchlorid, Aluminiumbromid oder Mischungen von Aluminiumchlorid oder Aluminiumbromid mit katalytischen Mengen an Bortrifluorid oder Eisen(III)chlorid.

Die Verwendung von Aluminiumchlorid als Lewis-Säure ist besonders hervorzuheben.

In manchen Fällen kann es auch von Vorteil sein, das neue Verfahren zusätzlich in Gegenwart von geringen Mengen an Borsäure durchzuführen.

Das erfindungsgemäße Verfahren kann sowohl in Anwesenheit als auch in Abwesenheit eines Verdünnungsmittels durchgeführt werden, wobei die Durchführung des Verfahrens in Gegenwart eines Verdünnungsmittels bevorzugt ist.

Geeignete Verdünnungsmittel sind z.B. Salze, insbesondere Alkali- oder Erdalkalihalogenide oder Ammoniumhalogenide, wie Lithiumchlorid, Lithiumbromid, Natriumchlorid, Natriumbromid, Magnesiumchlorid, Magnesiumbromid, Calciumchlorid, Calciumbromid, Ammoniumchlorid, Ammoniumbromid oder deren Mischungen.

Bevorzugt ist dabei die Verwendung von Natriumchlorid, Kaliumchlorid, Ammoniumchlorid oder deren Mischungen.

Besonders bevorzugt ist die Verwendung von Natriumchlorid, Kaliumchlorid oder deren Mischungen, wobei der Verwendung von Natriumchlorid besondere Bedeutung zukommt.

Im allgemeinen verwendet man je Mol an Lewis-Säure 0,25 bis 0,80 Mol des Verdünnungsmittels.

Günstige Ergebnisse erhält man bei der gleichzeitigen Verwendung von Aluminiumchlorid als Lewis-Säure

und Natriumchlorid, Kaliumchlorid oder deren Mischungen als Verdünnungsmittel. Es hat sich als vorteilhaft erwiesen, diese Komponenten dabei im Molverhältnis Aluminiumchlorid : Natriumchlorid oder Kaliumchlorid = 1,3:1 bis 4:1, vorzugsweise 1,7:1 bis 2,2:1, oder im Molverhältnis Aluminiumchlorid : Natriumchlorid : Kaliumchlorid = 3:1,25:1 bis 11,5:1,75:1, vorzugsweise 3,7:1,35:1 bis 5,4:1,45:1 anzuwenden.

Das Molverhältnis von Phthalsäureanhydrid II : Phenolderivat III beträgt üblicherweise 1:1 bis 10:1, vorzugsweise 1:1 bis 2,5:1.

Je Mol Phenolderivat III kommen im allgemeinen 2 bis 10 Mol, vorzugsweise 2 bis 3 Mol, Lewis-Säure zur Anwendung.

Das erfindungsgemäße Verfahren wird in einem selbstreinigenden Apparat mit Mischwirkung durchgeführt. Solche Apparate sind an sich bekannt und handelsüblich. Geeignete Reaktoren dieser Art sind z.B. der Kammerreaktor, der Kreislaufreaktor oder der Schneckenreaktor, wobei die Durchführung des erfindungsgemäßen Verfahrens im Schneckenreaktor bevorzugt ist.

Besonders hervorzuheben ist die Verwendung eines gleichläufigen, ineinandergreifenden zweiwelligen Schneckenkneters, wie er beispielsweise in H. Herrmann "Schneckenmaschinen in der Verfahrenstechnik", Seiten 120 bis 127, Springer Verlag, Berlin, 1972, beschrieben ist. Ein solcher Schneckenkneter besitzt als Misch- und Knetorgane Knetscheiben und arbeitet mit selbstreinigendem Dichtprofil.

Das neue Verfahren kann in diskontinuierlicher Arbeitsweise oder vorzugsweise in kontinuierlicher Arbeitsweise durchgeführt werden.

Die Umsetzung des Phthalsäureanhydrids II mit dem Phenolderivat III wird im allgemeinen bei Normaldruck und bei einer Temperatur von 120 bis 300°C, vorzugsweise 180 bis 250°C, insbesondere 220 bis 240°C, vorgenommen.

Die Reaktionszeit, d.h. die Verweilzeit im selbstreinigenden, dicht kämmenden Apparat mit Mischwirkung, beträgt üblicherweise 5 bis 1000 Sekunden, vorzugsweise 10 bis 600 Sekunden, und insbesondere 10 bis 100 Sekunden, wobei der Bereich von 10 bis 70 Sekunden besonders hervorzuheben ist.

Das Schergefälle im selbstreinigenden Apparat mit Mischwirkung beträgt üblicherweise 50 bis 20000 $sec^{-1}$, vorzugsweise 250 bis 3500 $sec^{-1}$. Der spezifische Energieeintrag während der Scherung beträgt in der Regel 0,01 bis 0,2 kWh/kg.

Da die Dosierung der Reaktionsteilnehmer sowohl in festem als auch in flüssigem Aggregatzustand möglich ist, bieten sich für die Durchführung des erfindungsgemäßen Verfahrens mehrere Varianten an:

- Die Zugabe von Phthalsäureanhydrid II, Phenolderivat III, Lewis-Säure und gegebenenfalls Verdünnungsmittel erfolgt als Gemisch oder getrennt voneinander, wobei alle Komponenten in festem Aggregatzustand vorliegen und im Reaktor in einer "Aufschmelzzone" in den flüssigen Aggregatzustand übergeführt werden.
- Lewis-Säure und gegebenenfalls Verdünnungsmittel werden vorgelegt und liegen in flüssigem Aggregatzustand vor. Dazu werden Phthalsäureanhydrid II und Phenolderivat III als Gemisch, in dem beide Komponenten in festem Aggregatzustand vorliegen, gegeben.
- Lewis-Säure und gegebenenfalls Verdünnungsmittel werden vorgelegt und liegen in flüssigem Aggregatzustand vor. Dazu werden Phthalsäureanhydrid II und Phenolderivat III als Gemisch, in dem beide Komponenten ebenfalls in flüssigem Aggregatzustand vorliegen, gegeben.
- Lewis-Säure und gegebenenfalls Verdünnungsmittel werden vorgelegt und liegen in flüssigem Aggregatzustand vor. Dazu werden Phthalsäureanhydrid II und Phenolderivat III entweder gleichzeitig aber räumlich getrennt voneinander oder aber zuerst Phthalsäureanhydrid II und anschließend Phenolderivat III gegeben, wobei Phthalsäureanhydrid II und Phenolderivat III dabei jeweils in flüssigem oder festem Aggregatzustand vorliegen können.

Wenn alle Reaktionsteilnehmer in den Reaktor dosiert sind, erfolgt, sofern dies nicht bereits durch die Überführung in den flüsigen Aggregatzustand geschehen ist, ihre Erwärmung auf die obengenannte Reaktionstemperatur und die Bildung des Zielprodukts findet nun statt.

Nach beendeter Umsetzung wird das Reaktionsgemisch mit wäßriger Säure behandelt, und das Zielprodukt I wird anschließend in an sich bekannter Weise abgetrennt, gewaschen und getrocknet.

Die Behandlung mit wäßriger Säure kann sowohl mit wäßrigen anorganischen Säuren, z.B. Salzsäure oder verdünnte Schwefelsäure, als auch mit wäßrigen organischen Säuren, z.ß. wäßrige Oxalsäure, vorgenommen werden. Die Verwendung von Salzsäure ist bevorzugt.

Führt man das erfindungsgemäße Verfahren mit Aluminiumchlorid als Lewis-Säure durch, so ist es zweckmäßig, den während der Umsetzung freiwerdenden Chlorwasserstoff in Wasser zu lösen und die so resultierende Salzsäure für die wäßrig-saure Behandlung zu verwenden.

Die Behandlung mit wäßriger Säure kann entweder diskontinuierlich oder vorzugsweise kontinuierlich durchgeführt werden.

Das erfindungsgemäße Verfahren zeichnet sich durch eine Reihe von Vorteilen aus. So ist die Reaktionszeit, d.h. die Verweilzeit im Reaktor, sehr kurz, wodurch ein selektiver Reaktionsverlauf gewährt ist. Die Zielprodukte fallen daher in hoher Reinheit an. Weiterhin ist auf die günstige Raum-Zeit-Ausbeute im neuen Verfahren hinzuweisen. Zusätzlich kann ein hoher Überschuß an einem der beiden Reaktionspartner vermieden werden, d.h. die Reaktanden werden annähernd in stöchiometrischem Verhältnis zur Reaktion gebracht. Schließlich kann auf die Anwesenheit eines organischen Lösungsmittels verzichtet werden.

Bei den Hydroxyanthrachinonen der Formel I handelt es sich um wertvolle Zwischenprodukte für die Herstellung von Anthrachinonfarbstoffen.

Die folgenden Beispiele sollen die Erfindung näher erläutern.

Beispiel 1

In einen zweiwelligen Schneckenkneter vom Typ ZSK (Fa. Werner und Pfleiderer, Stuttgart), dessen Innentemperatur 230°C betrug, wurden kontinuierlich 5 kg/h eines Gemisches, das aus 0,71 kg (6,45 Mol) Hydrochinon, 1,20 kg (8,10 Mol) Phthalsäureanhydrid, 2,58 kg (19,35 Mol) Aluminiumchlorid und 0,51 kg (8,73 Mol) Natriumchlorid (jeweils in festem Aggregatzustand) bestand, dosiert. Das Gemisch schmolz und reagierte ab. Der bei der Reaktion entstehende gasförmige Chlorwasserstoff (450 l/h) wurde aus dem Reaktor geleitet. Die Verweilzeit im Reaktor betrug 40 Sekunden. Der resultierende Aluminiumkomplex (4,3 kg/h) wurde 30 Minuten lang bei einer Temperatur von 80°C mit 12,9 kg 10 gew.-%iger Salzsäure hydrolysiert. Das Zielprodukt (1,4-Dihydroxyanthrachinon) wurde über eine Filterpresse abgesaugt mit 70 l Wasser neutral gewaschen und getrocknet. Ausbeute 1,0 kg/h (Ausbeute: 65 %; Reinheit: >95 %).

In analoger Weise wurden die in der folgenden Tabelle 1 aufgeführten Beispiele durchgeführt. Dabei gelten folgende Abkürzungen:

HC = Hydrochinon
PSA = Phthalsäureanhydrid

Tabelle 1

| Bsp. Nr. | PSA:HC | Molverhältnis $AlCl_3$:HC | $AlCl_3$:NaCl | Temp. | Verweil-zeit | Aus-beute | Rein-heit | 10 gew.-%ige Salzsäure pro Mol HC |
|---|---|---|---|---|---|---|---|---|
| 2 | 1,2:1 | 3,0:1 | 2,2:1 | 280°C | 40 sec | 82 % | 90 % | 1,5 kg |
| 3 | 1,2:1 | 3,0:1 | 2,2:1 | 230°C | 80 sec | 65 % | >95 % | 1,5 kg |
| 4 | 1,2:1 | 5,0:1 | 2,2:1 | 230°C | 40 sec | 80 % | >95 % | 2,0 kg |

In den in der folgenden Tabelle 2 aufgeführten Beispielen wurde jeweils eine Mischung von Aluminiumchlorid und Natriumchlorid (in flüssigem Aggregatzustand) im Schneckenreaktor (gemäß Beispiel 1) vorgelegt und Phthalsäureanhydrid und Hydrochinon (beide ebenfalls in flüssigem Aggregatzustand) gleichzeitig aber räumlich getrennt voneinander zudosiert.

Tabelle 2

| Bsp. Nr. | PSA:HC | Molverhältnis $AlCl_3$:HC | Molverhältnis $AlCl_3$:NaCl | Temp. | Verweil-zeit | Aus-beute | Rein-heit | 10 gew.-%ige Salzsäure pro Mol HC |
|---|---|---|---|---|---|---|---|---|
| 5 | 1,2:1 | 3,0:1 | 2,2:1 | 230°C | 40 sec | 90 % | >95 % | 2,0 kg |
| 6 | 1,2:1 | 3,0:1 | * | 230°C | 40 sec | 90 % | >95 % | 2,0 kg |
| 7** | 1,2:1 | 3,0:1 | 2,2:1 | 230°C | 40 sec | 90 % | >95 % | 2,0 kg |
| 8*** | 1,2:1 | 3,0:1 | 2,2:1 | 230°C | 40 sec | 85 % | >95 % | 2,0 kg |

*) Anstelle von Natriumchlorid wurde eine Mischung aus Natriumchlorid und Kaliumchlorid verwendet. Das Molverhältnis $AlCl_3$ : NaCl : KCl betrug 5,4:1,45:1.

**) Anstelle von Hydrochinon wurde Hydrochinondimethylether verwendet.

***) Anstelle von Hydrochinon wurde Hydrochinondiacetat verwendet.

Beispiel 9

Beispiel 9 wurde analog Beispiel 5 durchgeführt, jedoch wurde anstelle von Hydrochinon 4-Chlorphenol verwendet. Die Ausbeute von 1-Chlor-4-hydroxyanthrachinon betrug 90 % (Reinheit >95 %).

Beispiel 10

Beispiel 10 wurde analog Beispiel 1 durchgeführt, jedoch wurde anstelle von Phthalsäureanhydrid 1,2,3,4-Tetrachlorphthalsäureanhydrid verwendet. Bei einer Verweilzeit im Reaktor von 80 sec betrug die Ausbeute an 1,2,3,4-Tetrachlor-6,9-dihydroxyanthrachinon 92 % (Schmp. 240°C (Zers.)).

| | | | | | | | | | | |
|---|---|---|---|---|---|---|---|---|---|---|
| $C_{14}H_4Cl_4O_4$ | (378) | ber. | C | 44,4 | H | 1,1 | O | 16,9 | Cl | 37,6 |
| | | gef. | C | 43,8 | H | 1,0 | O | 17,1 | Cl | 37,8 |

$^{13}$C-NMR ([$D_6$]DMSO): δ = 113,5 (s; C-8a, C-5a), 129,0 (d; C-6, C-7), 131,5 (s; C-1, C-4), 132,3 (s; C-1a, C-4a), 139,7 (s; C-2, C-3), 155 (s; C-5, C-8), 183,5 ppm (s; C-9, C-10).

**Patentansprüche**

1. Verfahren zur Herstellung von Hydroxyanthrachinonen der Formel I

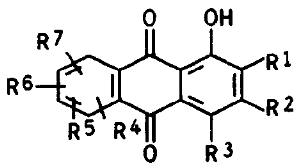

(I),

in der

$R^1$ und $R^2$ gleich oder verschieden sind und unabhängig voneinander jeweils Wasserstoff, Hydroxy, $C_1$-$C_4$-Alkyl oder Halogen oder $R^1$ und $R^2$ zusammen einen anellierten Benzolring, der gegebenenfalls substituiert ist, oder einen anellierten $C_1$-$C_4$-Cycloalkylring der gegebenenfalls substituiert ist,

$R^3$ Hydroxy, Amino oder Halogen und

$R^4$, $R^5$, $R^6$ und $R^7$ gleich oder verschieden sind und unabhängig voneinander jeweils Wasserstoff, Halogen, Hydroxy, $C_1$-$C_4$-Alkyl oder $C_1$-$C_5$-Alkanoyl bedeuten,

durch Umsetzung von Phthalsäureanhydriden der Formel II

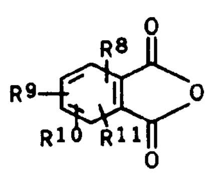

(II),

in der

$R^8$, $R^9$, $R^{10}$ und $R^{11}$ gleich oder verschieden sind und unabhängig voneinander jeweils Wasserstoff, Halogen, $C_1$-$C_4$-Alkyl, $C_1$-$C_5$-Alkanoyl, $C_1$-$C_4$-Alkoxy, $C_1$-$C_5$-Alkanoyloxy oder Sulfato bedeuten,

mit Phenolderivaten der Formel III

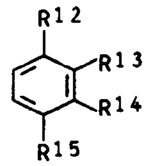

(III),

in der

$R^{12}$ Hydroxy, $C_1$-$C_4$-Alkoxy, $C_1$-$C_5$-Alkanoyloxy oder Sulfato,

$R^{13}$ und $R^{14}$ gleich oder verschieden sind und unabhängig voneinander jeweils Wasserstoff, Hydroxy, $C_1$-$C_4$-Alkoxy, $C_1$-$C_5$-Alkanoyloxy, $C_1$-$C_4$-Alkyl, Halogen oder Sulfato oder $R^{13}$ und $R^{14}$ zusammen einen anellierten Benzolring, der gegebenenfalls substituiert ist, oder einen anellierten $C_5$-$C_7$-Cycloalkylring, der gegebenenfalls substituiert ist, und

$R^{15}$ Hydroxy, $C_1$-$C_4$-Alkoxy, $C_1$-$C_5$-Alkanoyloxy, Sulfato, Halogen oder Mono- oder Di($C_1$-$C_5$-alkanoyl)amino bedeuten,

in Gegenwart einer Lewis-Säure als Katalysator und gegebenenfalls eines Verdünnungsmittels, dadurch gekennzeichnet, daß man die Umsetzung in einem selbstreinigenden Apparat mit Mischwirkung durchführt.

2. Verfahren nach Anspruch 1, dadurch gekennzeichnet, daß man Phthalsäureanhydride der Formel II, in der $R^8$, $R^9$, $R^{10}$ und $R^{11}$ gleich oder verschieden sind und unabhängig voneinander jeweils Wasserstoff oder Chlor bedeuten, mit Phenolderivaten der Formel III, in der $R^{12}$ Hydroxy, $C_1$-$C_4$-Alkoxy oder $C_1$-$C_5$-Alkanoyloxy, $R^{13}$ und $R^{14}$ jeweils Wasserstoff und $R^{15}$ Hydroxy, $C_1$-$C_4$-Alkoxy, $C_1$-$C_5$-Alkanoyloxy oder Chlor bedeuten, umsetzt.

3. Verfahren nach Anspruch 1, dadurch gekennzeichnet, daß man als Lewis-Säure Aluminiumchlorid, Aluminiumbromid oder Mischungen von Aluminiumchlorid oder Aluminiumbromid mit Bortrifluorid oder Eisen(III)chlorid verwendet.

4. Verfahren nach Anspruch 1, dadurch gekennzeichnet, daß man die Umsetzung in Gegenwart eines Verdünnungsmittels durchführt.

5. Verfahren nach Anspruch 1, dadurch gekennzeichnet, daß man als Verdünnungsmittel Natriumchlorid, Kaliumchlorid, Ammoniumchlorid oder deren Mischungen verwendet.

## Claims

1. A process for preparing a hydroxyanthraquinone of the formula I

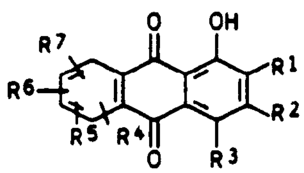

(I)

where

$R^1$ and $R^2$ are identical or different and each is independently of the other hydrogen, hydroxyl, $C_1$-$C_4$-alkyl or halogen or $R^1$ and $R^2$ together are a fused benzene ring which may be substituted or a fused $C_5$-$C_7$-cycloalkyl ring which may likewise be substituted,

$R^3$ is hydroxyl, amino or halogen and

$R^4$, $R^5$, $R^6$ and $R^7$ are identical or different and each is independently of the others hydrogen, halogen, hydroxyl, $C_1$-$C_4$-alkyl or $C_1$-$C_5$-alkanoyl,

by reacting a phthalic anhydride of the formula II

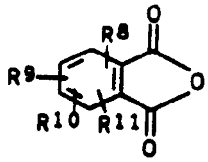

(II)

where

$R^8$, $R^9$, $R^{10}$ and $R^{11}$ are identical or different and each is independently of the others hydrogen, halogen, $C_1$-$C_4$-alkyl, $C_1$-$C_5$-alkanoyl, $C_1$-$C_4$-alkoxy, $C_1$-$C_5$-alkanoyloxy or sulfato,

with a phenol derivative of the formula III

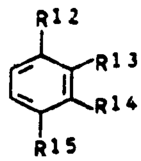

(III)

where

$R^{12}$ is hydroxyl, $C_1$-$C_4$-alkoxy, $C_1$-$C_5$-alkanoyloxy or sulfato,

$R^{13}$ and $R^{14}$ are identical or different and each is independently of the other hydrogen, hydroxyl, $C_1$-$C_4$-alkoxy, $C_1$-$C_5$-alkanoyloxy, $C_1$-$C_4$-alkyl, halogen or sulfato or $R^{13}$ and $R^{14}$ are together a fused benzene ring which may be substituted or a fused $C_5$-$C_7$-cycloalkyl ring which may likewise be substituted, and

$R^{15}$ is hydroxyl, $C_1$-$C_4$-alkoxy, $C_1$-$C_5$-alkanoyloxy, sulfato, halogen or mono- or di ($C_1$-$C_5$-alkanoyl)amino,

in the presence of a Lewis acid as catalyst and in the presence or absence of a diluent in a self-cleaning apparatus having a mixing effect.

**2.** A process as claimed in claim 1, wherein a phthalic anhydride of the formula II where $R^8$, $R^9$, $R^{10}$ and $R^{11}$ are identical or different and each is independently of the others hydrogen or chlorine is reacted with a phenol derivative of the formula III where $R^{12}$ is hydroxyl, $C_1$-$C_4$-alkoxy or $C_1$-$C_5$-alkanoyloxy, $R^{13}$ and $R^{14}$ are each hydrogen and $R^{15}$ is hydroxyl, $C_1$-$C_4$-alkoxy, $C_1$-$C_5$-alkanoyloxy or chlorine.

**3.** A process as claimed in claim 1, wherein the Lewis acid used is aluminum chloride, aluminum bromide or a mixture of aluminum chloride or aluminum bromide with boron tri fluoride or iron(III) chloride.

**4.** A process as claimed in claim 1, wherein the reaction is carried out in the presence of a diluent.

**5.** A process as claimed in claim 1, wherein the diluent used is sodium chloride, potassium chloride, ammonium chloride or a mixture thereof.

## Revendications

**1.** Procédé de préparation d'hydroxyanthraquinones de la formule I :

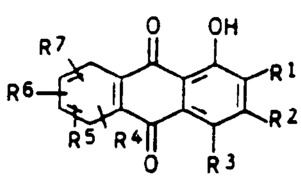

(I)

dans laquelle

R1 et $R^2$ sont identiques ou différents et représentent, chacun indépendamment l'un de l'autre, un atome d'hydrogène, un radical hydroxyle, un radical alkyle en $C_1$-$C_4$, ou un atome d'halogène, ou bien $R^1$ et $R^2$ forment ensemble un noyau benzénique condensé, qui est éventuellement substitué, ou un noyau cycloalkyle en $C_5$-$C_7$

condensé, qui est éventuellement substitué,

$R^3$ représente un radical hydroxyle, amino, ou un atome d'halogène, et

$R^4$, $R^5$, $R^6$ et $R^7$ sont identiques ou différents et représentent chacun indépendamment l'un de l'autre, un atome d'hydrogène, un atome d'halogène, un radical hydroxyle, alkyle en $C_1$-$C_4$, ou alcanoyle en $C_1$-$C_5$,

par la réaction d'anhydrides de l'acide phtalique de la formule II :

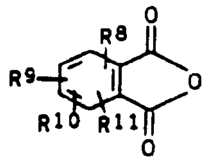

(II)

dans laquelle

$R^8$, $R^9$, $R^{10}$ et $R^{11}$ sont identiques ou différents et représentent chacun indépendamment l'un de l'autre, un atome d'hydrogène, un atome d'halogène, un radical alkyle en $C_1$-$C_4$, alcanoyle en $C_1$-$C_5$, alcoxy en $C_1$-$C_4$, alcanoyloxy en $C_1$-$C_5$, ou sulfato,

avec des dérivés du phénol de la formule III :

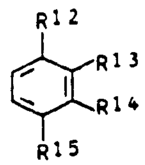

(III)

dans laquelle

$R^{12}$ représente un radical hydroxyle, alcoxy en $C_1$-$C_4$, alcanoyloxy en $C_1$-$C_5$, ou sulfato,

$R^{13}$ et $R^{14}$ sont identiques ou différents et représentent chacun indépendamment l'un de l'autre, un atome d'hydrogène, un radical hydroxyle, alcoxy en $C_1$-$C_4$, alcanoyloxy en $C_1$-$C_5$, alkyle en $C_1$-$C_4$, un atome d'halogène ou un radical sulfato, ou bien $R^{13}$ et $R^{14}$ forment ensemble un noyau benzénique condensé, qui peut éventuellement être substitué, ou un noyau cycloalkyle en $C_5$-$C_7$ condensé, qui peut éventuellement être substitué, et

$R^{15}$ représente un radical hydroxyle, alcoxy en $C_1$-$C_4$, alcanoyloxy en $C_1$-$C_5$, sulfato, un atome d'halogène, ou mono- ou di- (alcanoyl en $C_1$-$C_5$)-amino,

en présence d'un acide de Lewis servant de catalyseur et éventuellement d'un diluant, caractérisé en ce que l'on entreprend la réaction dans un appareil autonettoyeur à effet de mélange.

2. Procédé suivant la revendication 1, caractérisé en ce que l'on fait réagir des anhydrides de l'acide phtalique de la formule II, dans laquelle $R^8$, $R^9$, $R^{10}$ et $R^{11}$ sont identiques ou différents et représentent chacun indépendamment l'un de l'autre, un atome d'hydrogène ou de chlore, avec des dérivés du phénol de la formule III, dans laquelle $R^{12}$ représente un radical hydroxyle, alcoxy en $C_1$-$C_4$, ou alcanoyloxy en $C_1$-$C_5$, $R^{13}$ et $R^{14}$ représentent chacun un atome d'hydrogène et $R^{15}$ représente un radical hydroxyle, alcoxy en $C_1$-$C_4$, alcanoyloxy en $C_1$-$C_5$, ou un atome de chlore.

3. Procédé suivant la revendication 1, caractérisé en ce qu'à titre d'acide de Lewis, on utilise du chlorure d'aluminium, du bromure d'aluminium, ou des mélanges de chlorure d'aluminium ou de bromure d'aluminium avec du trifluorure de bore ou du chlorure de fer (III).

4. Procédé suivant la revendication 1, caractérisé en ce que l'on entreprend la réaction en présence d'un diluant.

5. Procédé suivant la revendication 1, caractérisé en ce que l'on utilise du chlorure de sodium, du chlorure de potassium, du chlorure d'ammonium, ou leurs mélanges, à titre de diluant.